# EUROPÄISCHE PATENTSCHRIFT

(11) **EP 4 228 549 B1**
(45) Veröffentlichungstag und Bekanntmachung des Hinweises auf die Patenterteilung: **09.10.2024**
(21) Anmeldenummer: 20838898.3
(22) Anmeldetag: 23.11.2020
(51) Int. Cl.: A61F 2/16

(54) **INJEKTOR FÜR INTRAOKULARLINSEN**
INJECTOR FOR INTRAOCULAR LENSES
INJECTEUR POUR LENTILLES INTRAOCULAIRES

(30) Priorität: 13.10.2020 DE 102020126789
(43) Veröffentlichungstag der Anmeldung: 23.08.2023
(73) Patentinhaber: OPHTHALMO Pro GmbH, 66386 Sankt Ingbert (DE)
(72) Erfinder: BAYER, Alexander, 40472 Düsseldorf (DE)
(74) Vertreter: Walther Bayer Faber Patentanwälte PartGmbB
(86) Internationale Anmeldenummer: PCT/EP2020/083025
(87) Internationale Veröffentlichungsnummer: WO 2022/078617

(56) Entgegenhaltungen:
- EP-A1- 2 649 962
- EP-A1- 3 476 375
- US-A1- 2009 112 223

## Beschreibung

Die Erfindung betrifft einen Injektor zur Implantation einer Intraokularlinse in ein menschliches oder tierisches Auge, aufweisend einen Grundkörper, in den rückseitig ein länglich ausgebildetes Betätigungselement abschnittsweise hineinragt und das in einer Betätigungsachse beweglich geführt ist, und wobei eine Bewegung des Betätigungselementes in den Grundkörper wahlweise mittels einer Schubbetätigung entlang der Betätigungsachse oder mittels einer Schraubbetätigung um die Betätigungsachse herum erzeugbar ist, und wobei das Betätigungselement wenigstens auf einem Abschnitt ein Außengewinde aufweist.

### STAND DER TECHNIK

Injektoren zur Implantation von Intraokularlinsen in ein menschliches oder tierisches Auge sind hinreichend bekannt. Eine Intraokularlinse kann hierfür entweder über eine Linsenkartusche mit einer in dieser eingebrachten Intraokularlinse kurz vor dem Einsetzen in das Auge in den Injektor eingebracht werden, um nach anschließender Zugabe eines Viskoelastikums die Intraokularlinse aus einer vorderseitigen Austriebsdüse in die hintere Augenkammer des Auges auszutreiben. Die Austriebsdüse bildet das offene Ende eines Linsenführungsteils des Injektors, der vorderseitig am Grundkörper angeordnet ist.

Im Grundkörper ist ein Kolben entlang der Betätigungsachse beweglich geführt, und der Kolben, der mit dem Betätigungselement in der Betätigungsachse voreinander liegend in Verbindung steht, kann bei einem Vorschub des Betätigungselementes und damit übertragen auf den Kolben in Richtung zur Austriebsdüse mit der Intraokularlinse in Kontakt gelangen und diese durch die Austriebsdüse austreiben.

Um den Kolben innerhalb des Grundkörpers des Injektors und weiter voranschreitend in Richtung zur Austriebsdüse auch durch das Linsenführungsteil hindurch kontrolliert zu bewegen, ist das Betätigungselement vorgesehen, das so in den Grundkörper hineinragt, dass bei einem Vorschub des Betätigungselementes in der Betätigungsachse der Kolben erst die Intraokularlinse kontaktiert und im weiteren Verlauf gemeinsam mit der Intraokularlinse in Richtung zur Austriebsdüse bewegt werden kann, bis die Intraokularlinse in die hintere Augenkammer ausgetrieben ist.

Injektoren der neueren Bauart werden als Einweginjektoren verwendet und nach dem einmaligen Gebrauch und dem Austrieb der voreingelegten Intraokularlinse entsorgt. Dieses System der Injektoren wird allgemein als Pre-Load System bezeichnet.

Das Einschieben des Betätigungselementes in eine hintere Öffnung im Grundkörper erfolgt dabei in der Regel manuell durch den Bediener, insbesondere durch den Ophthalmologen. Dabei sind Injektoren bekannt, die zwischen zwei Betriebsmodi umschaltbar sind und die in einem ersten Betriebsmodus eine reine Schubbewegung ermöglichen, die manuell in das Betätigungselement eingebracht wird, und die in einem zweiten Betriebsmodus eine Drehbewegung zum Austrieb der Intraokularlinse ermöglichen, die in das gleiche Betätigungselement eingebracht wird, um damit das Betätigungselement entlang der Betätigungsachse zu bewegen. Insofern sind Injektoren neuerer Bauart in der Lage, sowohl eine Schubbetätigung als auch eine Schraubbetätigung zu erlauben, wofür am Injektor eine entsprechende Vorrichtung zur Umschaltung zwischen den Betriebsmodi vorgesehen wird.

Beispielsweise offenbart die EP 3 476 375 A1 eine Ausgestaltung eines Injektors zur Implantation einer Intraokularlinse, und das Betätigungselement kann entweder durch ein direktes rückseitiges Einschieben in den Grundkörper eingefahren werden, oder es wird ein Stellelement betätigt, in das eine Schraubbewegung eingeleitet wird, wobei das Stellelement eine direkte Gewindeverbindung mit einem Außengewinde des Betätigungselementes aufweist. Das bedeutet, dass wenn das Betätigungselement manuell in den Grundkörper hineingedrückt wird, sich das Stellelement frei dreht. Wird das Betätigungselement also wahlweise durch rückseitiges Drücken auf das Betätigungselement oder durch ein Drehen des Stellelementes in den Grundkörper hineingetrieben, so kann dieses in Kontakt mit dem Kolben die Intraokularlinse vorderseitig aus der Austriebsdüse austreiben. Das Stellelement muss zur Umsetzung diese Wirkprinzips eine direkte Gewindeverbindung mit dem Betätigungselement aufweisen.

Die WO 2019/195951 A1 offenbart eine weitere Ausgestaltung eines Injektors zur Implantation einer Intraokularlinse in ein menschliches oder tierisches Auge, aufweisend einen Grundkörper, in den rückseitig ein länglich ausgebildetes Betätigungselement abschnittsweise hineinragt und in einer Betätigungsachse beweglich geführt ist, und wobei eine Bewegung des Betätigungselementes in den Grundkörper wahlweise mittels einer Schubbetätigung des Betätigungselementes entlang der Betätigungsachse oder mittels einer Schraubbetätigung des Betätigungselementes um die Betätigungsachse erzeugbar ist, wofür das Betätigungselement wenigstens auf einem Abschnitt ein Außengewinde aufweist. Um zwischen der Schubbetätigung und der Schraubbetätigung des Betätigungselementes umzuschalten, sind zwei sich gegenüberliegende ein- und ausklappbare Flügelgriffe vorgesehen, wobei durch die ausgeklappte Position der Flügelelemente der Betriebsmodus auf eine Schubbetätigung und in einer eingeklappten Position der Flügelelemente der Betriebsmodus auf eine Schraubbetätigung umgeschaltet werden kann.

Im Betriebsmodus der Schubbetätigung können dabei die ein- und ausklappbaren Flügelgriffe als Fingergriffe dienen, sodass der Ophthalmologe den Injektor an den Flügelgriffen zwischen Zeigefinger und Mittelfinger greifen kann, um schließlich mit dem Daumen rückseitig das Betätigungselement in den Grundkörper hineinzuschieben. Sind die Flügelgriffe eingeklappt, kann das Betätigungselement in den Grundkörper eingeschraubt werden, indem der Grundkörper in einer ersten Hand des Ophthalmologen gehalten wird und das Betätigungselement mit der zweiten Hand des Ophthalmologen eingeschraubt wird.

Die Einstellung des Injektors entweder für die Schubbetätigung oder für die Schraubbetätigung erfolgt in der Regel durch eine assistierende Person, sodass anschließend der Ophthalmologe den Injektor mit dem voreingestellten Betriebsmodus anwendet, indem die Austriebsdüse am vorderen Linsenführungsteil in den Augapfel eingeführt wird, und indem anschließend die Betätigung des Betätigungselementes erfolgt, bis die Intraokularlinse in die hintere Augenkammer eingetrieben wurde.

### OFFENBARUNG DER ERFINDUNG

Aufgabe der Erfindung ist die Weiterbildung eines Injektors zur Implantation einer Intraokularlinse in ein menschliches Auge, wobei der Injektor eine möglichst einfache Umschaltbarkeit zwischen der Schubbetätigung und der Schraubbetätigung des Betätigungselementes zum Vortrieb der Intraokularlinse aufweisen soll. Insbesondere soll eine Umschaltung des Betriebsmodus zwischen der Schubbetätigung und der Schraubbetätigung so möglich sein, dass der Injektor im Übrigen auf gleiche Weise zu benutzen ist, wobei jedoch die Umschaltung zwischen der Schubbetätigung und der Schraubbetätigung unmittelbar vor der Benutzung des Injektors einstellbar sein sollte.

Diese Aufgabe wird ausgehend von einem Injektor gemäß Anspruch 1 gelöst. Vorteilhafte Weiterbildungen der Erfindung sind in den abhängigen Ansprüchen angegeben.

Die Erfindung schließt die technische Lehre ein, dass am Grundkörper ein Stellelement um die Betätigungsachse verdrehbar oder in der Betätigungsachse verschiebbar angeordnet ist, wobei das Stellelement wenigstens einen Federarm aufweist, der an einer zum Außengewinde weisenden Innenfläche eine Gewindeeingriffsstruktur aufweist, wobei am Grundkörper eine Innenfläche ausgebildet ist, die mit einer Außenfläche am Federarm einen Wirkkontakt bildet, wobei die Innenfläche und/oder die Außenfläche unter einem Winkel zur Betätigungsachse geneigt ausgebildet ist oder wobei die Innenfläche in einer Umfangsrichtung um die Betätigungsachse wenigstens abschnittsweise einen sich ändernden Innenradius aufweist, sodass die Verdrehung oder die Verschiebung des Stellelementes eine elastische Verformung des Federarms zur Betätigungsachse hin und von der Betätigungsachse weg erzeugbar ist und sodass die Gewindeeingriffsstruktur mit dem Außengewinde wahlweise in Eingriff oder außer Eingriff bringbar ist.

Kerngedanke der Erfindung ist ein Stellelement am Gehäuse des Injektors, das manuell zwischen zwei Stellpositionen verdreht oder verschoben werden kann, und in einer ersten Stellposition des Stellelements wird ein Eingreifen der Gewindeeingriffsstruktur am Federarm in das Außengewinde des Betätigungselementes für die Schraubbetätigung erzeugt, und in einer zweiten Stellposition des Stellelementes wird die Gewindeeingriffsstruktur am Federarm aus dem Außengewinde des Betätigungselementes außer Eingriff gebracht, sodass schließlich die Schubbetätigung des Injektors eingestellt ist.

Wird also das Stellelement in die zweite Stellposition verdreht oder verschoben, so kann der Federarm oder es können mehrere Federarme, insbesondere zwei sich gegenüberliegende Federarme, von selbst in Bezug auf die Betätigungsachse elastisch wieder nach außen federn, sodass die Gewindeeingriffsstruktur das Außengewinde des Betätigungselementes freigibt. Die Wirkverbindung zwischen der Innenfläche am Grundkörper und dem Federarm wird erfindungsgemäß durch einen Wirkkontakt erzeugt, der so ausgebildet ist, dass bei einer axialen oder rotatorischen Verlagerung des Stellelementes am Grundkörper eine Bewegung des Federarms am Stellelement erzeugt wird, die mit Bezug auf die Betätigungsachse in radialer Richtung erfolgt, nämlich dass der Federarm zur Betätigungsachse und damit zum Außengewinde des Betätigungselementes hin und von diesem wieder wegbewegt werden kann.

Die axiale Richtung beschreit dabei eine sich in der Betätigungsachse erstreckende Richtung und eine radiale Richtung beschreibt eine senkrecht auf die Betätigungsachse stehende Richtung. Insofern stehen die axiale und die radiale Richtung im vorliegenden Sinne senkrecht aufeinander.

Um die Bewegung im Federarm durch eine Bewegung des Stellelementes zu erzeugen, weist nach einer ersten Ausführungsform wenigstens eine am Wirkkontakt beteiligte Fläche, insofern entweder die Innenfläche im oder am Grundkörper und/oder die Außenfläche am Federarm, eine Neigung auf, die einen Winkel zum Verlauf der Betätigungsachse besitzt. Der Winkel kann dabei abgetragen werden zwischen der Betätigungsachse und einem Vektor, der in der beteiligten Fläche liegt, also der Innenfläche und/oder der Außenfläche, wobei der Vektor durch den Kontaktpunkt oder die Kontaktlinie zwischen der Innenfläche und der Außenfläche verläuft und einen Winkelwert aufweist, der größer als 0° und kleiner als 90° beträgt. Selbstverständlich kann die Innenfläche und/oder die Außenfläche des Stellelementes beziehungsweise des Federarms auch gewölbt oder auf sonstige Weise abweichend von einer Ebene ausgestaltet sein, ohne dabei vom erfindungsgemäßen Gedanken eines Wirkkontaktes unter einem Winkel abzuweichen.

Die Neigung der beteiligten Fläche kann in der Innenfläche am Grundkörper ausgebildet sein, während die Außenfläche des Federarms gerade, das heißt in Verlaufsrichtung etwa parallel zur Betätigungsachse ohne eine Winkelneigung verlaufend ausgebildet ist. Mit dem gleichen Effekt kann auch die Außenfläche des Federarms unter dem Winkel zur Betätigungsachse ausgeführt sein, während die Innenfläche gerade und parallel zur Betätigungsachse verlaufend ausgebildet ist, insbesondere zylindrisch um die Betätigungsachse verlaufend, dabei stößt die schräge Außenfläche des Federarms beispielsweise gegen eine Kante in der Innenfläche und bewirkt so bei einer axialen Verlagerung ebenfalls eine radiale elastische Bewegung.

Selbstverständlich funktioniert das erfindungsgemäße Wirkprinzip zwischen dem Grundkörper und dem Federarm besonders gut, wenn sowohl die Innenfläche als auch die Außenfläche eine Neigung oder eine Wölbung aufweisen, die insbesondere jeweils den gleichen Winkel zur Betätigungsachse einschließen. In jedem Falle der Ausgestaltung der Innenfläche und der Außenfläche entsteht jedoch ein Wirkkontakt, der unter einem Winkel relativ zur Betätigungsachse geneigt ist. Das Stellelement muss nicht einteilig ausgeführt sein, und dieses kann auch mehrteilig sein, ebenso kann auch der Grundkörper einteilig oder mehrteilig ausgeführt sein. Insofern kann die Innenfläche beispielsweise auch innerhalb eines Ringkörpers ausgebildet sein, der in einem Innendurchgang im Grundkörper nur angeordnet oder befestigt wird, wobei der Ringkörper dabei im Sinne der vorliegenden Erfindung dennoch Teil des Grundkörpers ist. Damit ist eine Innenfläche auch dann am Grundkörper ausgebildet, wenn der Grundkörper mehrteilig ausgeführt ist und die Innenfläche ist nicht unmittelbar an einem Hauptteil des Grundkörpers ausgebildet sondern etwa an einem Zusatzelement oder dergleichen.

Das Stellelement weist im Sinne der Erfindung etwa auch dann ein Federelement auf, wenn dieses nicht einteilig und/oder materialeinheitlich mit dem Körper des Stellelementes ausgeführt ist sondern nur an diesem angeordnet oder mit dem Stellelement bewegbar oder wirkverbunden ist, aber ansonsten beispielsweise vereinzelbar ist.

Beispielsweise ist das Stellelement als Schiebeelement ausgeführt und im oder am Grundkörper beweglich angeordnet. Auch oder alternativ kann das Stellelement ringförmig oder hülsenförmig ausgeführt sein und das Betätigungselement teilumfänglich oder vollumfänglich umschließen und am Grundkörper verschiebbar oder verdrehbar sein.

Die Innenfläche ist am Grundkörper so angeordnet, dass bei einer axialen Verlagerung des Stellelementes in Form eines Schiebeelementes und damit auch bei einer axialen Verlagerung des wenigstens einen am Stellelement angeordneten Federarmes relativ zum Grundkörper sich die Verlagerung des Federarmes auch relativ zur Innenfläche ergibt. So kann das Prinzip des Wirkkontaktes unter einem Winkel zur Betätigungsachse insofern auch bei einem Schiebeelement genutzt werden, das nicht verdreht werden muss und beispielsweise an nur einer Umfangsposition im oder am Gehäuse verschoben werden kann. Im bevorzugten Fall ist das Stellelement aber ringförmig oder hülsenförmig ausgeführt und das Betätigungselement wird teilumfänglich oder vollumfänglich vom Stellelement umschlossen.

Mit weiterem Vorteil können wenigstens die am Wirkkontakt beteiligten Flächen eine Beschichtung aufweisen, die ein besonders günstiges Abgleiten der Flächen aufeinander ermöglicht, sodass insofern die Innenfläche undloder die Außenfläche die Beschichtung aufweist.

Vorteilhafterweise weist der Grundkörper einen Aufnahmeabschnitt mit einem Durchgang auf, durch den sich das Betätigungselement hindurch erstreckt und wobei das Stellelement am oder auf dem Aufnahmeabschnitt entlang der Betätigungsachse axial beweglich und/oder um die Betätigungsachse drehbeweglich aufgenommen ist. Das Stellelement kann auch innenseitig in den Aufnahmeabschnitt eingebracht sein, insbesondere wenn dieser hülsenförmig ausgebildet ist, wobei eine manuelle Verstellbarkeit von der Außenseite für den Benutzer gewahrt bleiben muss. Mit besonderem Vorteil kann das Stellelement einen Hülsenabschnitt aufweisen, der außenseitig auf dem Aufnahmeabschnitt verdrehbar oder verschiebbar aufgebracht ist, und der Federarm oder die Federarme umgreifen das offene Ende des Aufnahmeabschnittes und ragen innenseitig in diesen hinein, insbesondere so weit, dass das Ende des Federarmes oder die Enden der Federarme in den Ringspalt zwischen dem Betätigungselement und der Innenfläche in den Grundkörper hineinragen.

Vorteilhafterweise ist die Innenfläche so am Grundkörper ausgebildet, dass diese sich innenseitig im Aufnahmeabschnitt des Grundkörpers befindet. Damit ergibt sich eine vorteilhafte Geometrie, denn die Federarme müssen nicht unnötig lang ausgeführt werden. Eine Anordnung der Innenfläche zur Wechselwirkung mit dem oder mit den Federarmen ist im Rahmen der Erfindung allerdings auch innenseitig in einem weiter zur Vorderseite des Injektors liegenden Hohlraum im Grundkörper möglich.

Der wenigstens eine Federarm ist damit so am Stellelement angeordnet, dass sich der Federarm zumindest abschnittsweise zwischen das Betätigungselement und der Innenfläche im Grundkörper hinein erstreckt und/oder sich in den Aufnahmeabschnitt hinein erstreckt.

Gemäß einer ersten möglichen Ausführungsform bildet die Innenfläche wenigstens abschnittsweise einen Innenkegel im Grundkörper. Der Effekt des radialen Zufahrens des wenigstens einen Federarmes und seines elastischen radial nach außen gerichteten Rückfederns wird dabei erzeugt durch eine axiale Verlagerung des Stellelementes und damit des wenigstens einen Federarmes entlang der Betätigungsachse, wobei die axiale Verlagerung des Stellelementes beispielsweise mittels einer Drehbewegung des Stellelementes auf dem Aufnahmeabschnitt erzeugt werden kann. Insofern drehen die Federarme im Innenkegel mit einer Drehung des Stellelementes um die Betätigungsachse mit, was der Funktion der radialen Bewegung der Federarme aber nicht entgegensteht.

Gemäß einer zweiten möglichen Ausführungsform ist die Innenoberfläche mit dem sich in Umfangsrichtung ändernden Innenradius auf einem Umfangssegment um die Betätigungsachse im Grundköper ausgebildet, wobei das Umfangssegment sich auf einem Umfang mit einem Umfangswinkel von 360° oder auf einem Umfangswinkel von 180° oder auf einem Umfangswinkel von 120° oder auf einem Umfangswinkel von 90° erstreckt. Entsprechend der Anzahl der Umfangssegmente ist es vorteilhaft, jedem Umfangssegment einen Federarm zuzuordnen. So ist insbesondere vorgesehen, dass wenigstens ein Federarm, zwei sich gegenüberstehende Federarme, drei, vier oder mehr Federarme am Stellelement angeordnet sind.

Durch eine Innenoberfläche mit dem sich in Umfangsrichtung, das bedeutet sich über dem Umfang ändernden Innenradius wird bei einer Verdrehung des Stellelementes in eine erste Stellposition der wenigstens eine Federarm radial nach innen gedrückt, wenn der Innenradius an dieser Stelle kleiner ist, sodass die Gewindeeingriffsstruktur mit dem Außengewinde in Eingriff gelangt. Hierfür steht die Außenfläche des Federarms, insbesondere rückseitig der innenliegenden Gewindeeingriffsstruktur, mit der Innenoberfläche im Grundkörper in Kontakt, und die Innenoberfläche zieht sich hierfür gewissermaßen um den Federarm zu, da die Federarme mit einer Drehung des Stellelementes innerhalb der Innenoberfläche mitverdrehen und mit verschiedenen Umfangspositionen der Innenoberfläche und folglich mit verschiedenen Innenradien in Kontakt gelangen. Bei einem Rückdrehen in entgegengesetztem Drehsinn gelangen die Federarme wieder mit einem größeren Innenradius der Innenfläche in Kontakt, sodass der oder die Federarme selbstständig und elastisch wieder nach außen zurück federn. Die Federarme sind dabei mit der Drehung des Stellelementes mitdrehbar, sodass diese bei einer Verdrehung des Stellelementes im Beriech der Innenoberfläche am Grundkörper ebenfalls um die Betätigungsachse verdrehen. Die Federarme können dabei auch nur mittelbar an dem Stellelement angeordnet sein.

Das Stellelement ist insbesondere gewindelos ausgeführt. Auf diese Weise kann der Injektor mit einem einfachen Stellelement in Form eines Drehringes oder eines Drehkranzes zwischen den Betriebsmodi der Schubbetätigung und der Schraubbetätigung umgeschaltet werden, ohne dass außenseitig am Injektor ein Element umgeklappt und damit die Handhabung verändert werden muss. Das Stellelement kann beschriftet sein, beispielsweise mit "Push" und "Screw", und es können Pfeile auf dem Stellelement aufgebracht sein, sodass der Benutzer unschwer und unmittelbar erkennt, in welcher Richtung das Stellelement um die Betätigungsachse in Anordnung am Grundkörper verdreht werden muss, um wahlweise die Schubbetätigung oder die Schraubbetätigung zu aktivieren. Der Kontakt zwischen dem Federarm und dem Gehäuse im Bereich der Innenoberfläche muss im Rahmen einer möglichen erfindungsgemäßen Ausgestaltung kein direkter Kontakt sein und es können auch Elemente zwischengeordnet sein, die den kraftübertragenden Kontakt im Sinne der Erfindung ebenfalls ermöglichen.

Zwischen dem Aufnahmeabschnitt und dem Stellelement sind vorteilhafterweise wenigstens eine Nut und ein Zapfen ausgebildet, wobei der Zapfen in der Nut geführt ist. Vorzugsweise ist etwa im Außenumfang des Aufnahmeabschnittes die wenigstens eine Nut eingebracht, wobei innenseitig im Stellelement der wenigstens eine Zapfen ausgebildet ist, der in der Nut geführt ist. Insbesondere kann das Stellelement mittels einer Gewindeverbindung auf dem Aufnahmeabschnitt angeordnet sein, sodass mit einer Verdrehung des Stellelementes um die Betätigungsachse das Stellelement eine Bewegung entlang der Betätigungsachse ausführt.

Ist die Innenoberfläche mit einem Umfangssegment gemäß der zweiten möglichen Ausführungsform mit einem sich ändernden Innenradius ausgeführt, so ist vorzugsweise wenigstens eine Nut um die Betätigungsachse an einer Axialposition nur umlaufend zwischen dem Aufnahmeabschnitt und dem Stellelement ausgebildet, da bei dieser Variante das Stellelement und damit der wenigstens eine Federarm nicht axial in der Betätigungsachse verlagert sondern nur um diese verdreht werden muss.

Ist das Stellelement ringförmig oder hülsenförmig ausgeführt, ist dieses nicht zwingend aber bevorzugt so am Grundkörper angeordnet, dass die Rotationsachse des ringförmigen oder hülsenförmigen Stellelementes mit der Betätigungsachse zusammenfällt.

Dabei ist die Handhabung des Injektors mit einem verdrehbaren Stellelement in der Regel einfacher und für die Handhabung angenehmer als das axiale Schieben eines Schiebeelementes zwischen zwei Rastpositionen, wobei es jedoch auch denkbar ist, dass ein ringförmig oder hülsenförmig ausgestaltetes Stellelement ebenfalls in der Betätigungsachse am Grundkörper verschiebbar ist, dieses insofern also zwischen zwei Rastpositionen verlagert werden kann.

Schließlich ist für die erste mögliche Ausführungsform vorgesehen, dass das Stellelement eine Schiebe-Dreh-Beweglichkeit in Anordnung am Grundkörper aufweist, woraus die am meisten bevorzugte Variante erzielt wird, nämlich wenn das Stellelement mit einer gewindeartigen Führung zwischen dem Stellelement und dem Grundkörper am Grundkörper bei einer Verdrehung axial verlagert werden kann. Wird das Stellelement manuell verdreht, und erfolgt die Wirkverbindung zwischen dem Stellelement und dem Grundkörper über eine entsprechende gewindeartige Führung, und so kann die axiale Verlagerung des Stellelementes in der Betätigungsachse auf diese Weise sehr komfortabel erzielt werden.

Es ist auch denkbar, dass das Stellelement in einer festen Axialposition verdrehbar am Grundkörper angeordnet ist, und es kommt trotzdem die Innenfläche in Form des Innenkegels zum Einsatz. Diese Ausgestaltung wird möglich, wenn zur axialen Verlagerung des oder der Federarme eine Anordnung des wenigstens einen Federarms am Stellelement so entkoppelt ausgeführt ist, dass diese beispielsweise ebenfalls über eine eigene Kulisse oder dergleichen vom Stellelement bei einer Drehung des Stellelementes axial bewegt werden, ähnlich wie bei einem Kugelschreiber, der bei einer Verdrehung eines vorderen oder hinteren Teils eine Mine einfahren oder ausfahren kann. Eine solche Wirkverbindung wird vorliegend auch von einer Anordnung des wenigstens einen Federarms an dem Stellelement umfasst.

Schließlich kann der Grundkörper einen Flügelgriff aufweisen, wobei sich der Aufnahmeabschnitt rückseitig des Flügelgriffes als Fortsatz anschließt. Der Aufnahmeabschnitt, der Flügelgriff und der sonstige, übrige Grundkörper können lediglich bevorzugt aber nicht einschränkend einteilig und materialeinheitlich ausgestaltet und insbesondere im Spritzguss hergestellt sein.

Weiterhin ist es vorteilhaft, wenn der Injektor ein Aufnahmemittel für eine Linsenkartusche aufweist, wobei in die Linsenkartusche eine Intraokularlinse eingebracht ist und wobei die Linsenkartusche mit der Intraokularlinse in das Aufnahmemittel einsetzbar ist. Derartige Injektoren können insbesondere mehrfach verwendbar sein, sofern diese resterilisierbar sind. Jedoch kann auch ein mit einer Kartusche beladbarer Injektor als Wegwerf-Einwegartikel ausgeführt sein.

Der Injektor kann auch als sogenanntes Pre-Load-System ausgeführt sein, gemäß dem der Injektor eine im Grundkörper und/oder in einem vorderseitig am Grundkörper angeordneten Linsenführungsteil ausgebildete Aufnahmekammer aufweist, in der eine Intraokularlinse bereits herstellerseitig eingelegt ist, sodass der Injektor mit der eingelegten Intraokularlinse eine einzeln handhabbare und handelbare Einheit bildet. Der erfindungsgemäße Injektor kann insofern als Pre-Load-System oder als Kartuschensystem funktionieren.

Schließlich ist im Grundkörper ein Kolben aufgenommen, der vorteilhafterweise über einen Drehübertrager mit dem Betätigungselement axial verschiebbar ist. Insbesondere dann, wenn die Schraubbetätigung des Injektors eingeschaltet wird, muss das Betätigungselement die Drehbewegung ausführen, während der Kolben lediglich eine Linearbewegung in der Betätigungsachse ausführt, ohne sich dabei in der Betätigungsachse zu drehen.

Der Grundkörper ist vorteilhafterweise mit dem Aufnahmeabschnitt und mit dem Flügelgriff einteilig und aus nur einem Kunststoffkörper gebildet, wobei das vorderseitig am Grundkörper angeordnete Linsenführungsteil mit einer spitzenseitigen Austriebsdüse zum Austrieb der Intraokularlinse ausgebildet und an den Grundkörper insbesondere lösbar angeordnet ist.

### BEVORZUGTE AUSFÜHRUNGSBEISPIELE DER ERFINDUNG

Weitere, die Erfindung verbessernde Maßnahmen werden nachstehend gemeinsam mit der Beschreibung bevorzugter Ausführungsbeispiel der Erfindung anhand der Figuren näher dargestellt. Es zeigt:
- Figur 1: eine perspektivische Ansicht des Injektors zur Implantation einer Intraokularlinse in ein menschliches oder tierisches Auge,
- Figur 2: eine Explosionsdarstellung der wesentlichen Bestandteile des Injektors gemäß Figur 1,
- Figur 3: eine quergeschnittene Ansicht des Injektors mit einer Innenfläche in dessen Grundkörper, die gemäß einem ersten Ausführungsbeispiel einen Innenkegel bildet;

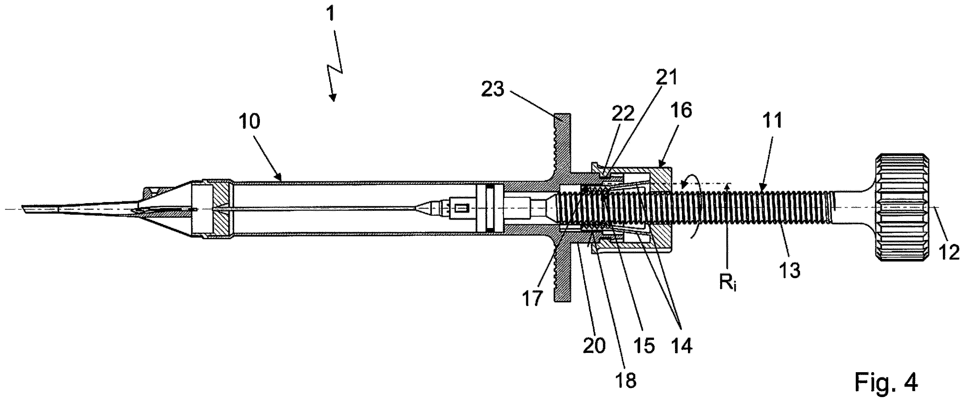

- Figur 4: eine quergeschnittene Ansicht des Injektors mit einer Innenfläche in dessen Grundkörper, die gemäß einem zweiten Ausführungsbeispiel um die Betätigungsachse herum einen in Umfangsrichtung sich ändernden Innenradius aufweist,
- Figur 5: eine Querschnittsansicht des Injektors im Bereich der Innenfläche, die gemäß einem zweiten Ausführungsbeispiel einen über ihrem Umfang um die Betätigungsachse veränderlichen Innenradius aufweist und wobei die gezeigten Federarme nicht in das Außengewinde des Betätigungselementes eingreifen und
- Figur 6: die Querschnittsansicht des Injektors im Bereich der Innenfläche gemäß Figur 5, wobei die gezeigten Federarme in das Außengewinde des Betätigungselementes eingreifen.

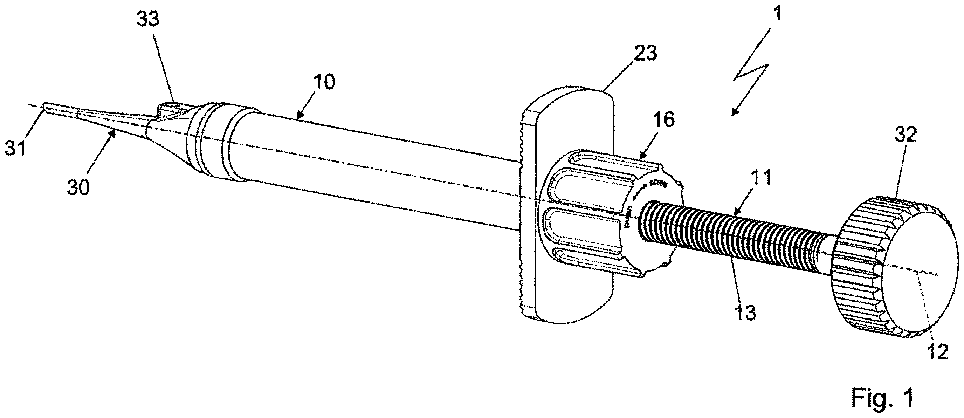

Figur 1 zeigt eine perspektivische Ansicht eines Injektors 1, der zur Implantation einer Intraokularlinse in die hintere Augenkammer eines menschlichen oder tierischen Auge dient. Der Injektor 1 weist als wesentliches Strukturbauteil einen Grundkörper 10 auf, und in diesen ist rückseitig ein länglich ausgebildetes Betätigungselement 11 mit einem endseitigen Griffstück 32 teilweise hineingeführt. Das Betätigungselement 11 besitzt über seiner wesentlichen Länge einen Abschnitt mit einem Außengewinde 13, und das Betätigungselement 11 ist abschnittsweise rückseitig in den Grundkörper 10 so eingeführt, dass das Griffstück 32 an einem freien Ende des Betätigungselementes 11 ausgebildet ist.

Vorderseitig am Grundkörper 10 ist ein Linsenführungsteil 30 angeordnet, in dem in nicht näher gezeigter Weise eine Intraokularlinse aufgenommen ist. Der wesentliche Bestandteil des Grundkörpers 10 ist etwa zylinderförmig oder für die Handhabung ergonomisch für eine menschliche Hand ausgestaltet und weist eine längliche Erstreckung auf, und der Injektor 1 mit dem Grundkörper 10, mit dem vorderseitigen Linsenführungsteil 30 und mit dem rückseitig eingeführten Betätigungselement 11 erstreckt sich längs in einer Betätigungsachse 12. Die Betätigungsachse 12 bildet dabei zugleich die Schubachse und die Rotationsachse für das wahlweise verschiebbare oder drehbare Betätigungselement 11.

Zur verbesserten Handhabung befindet sich am hinteren Teil des Grundkörpers 10 ein Flügelgriff 23, sodass der Injektor 1 mit dem Flügelgriff 23 zwischen dem Zeigefinger und dem Mittelfinger gehalten werden kann, während der Benutzer mit dem Daumen rückseitig auf das Griffstück 32 drücken kann, wenn die Schubbetätigung eingestellt ist.

Rückseitig zum Flügelgriff 23 schließt sich ein erfindungsgemäßes Stellelement 16 an, das um die Betätigungsachse 12 drehbar oder in der Betätigungsachse 12 verschiebbar am Grundkörper 10 aufgenommen ist, und das Stellelement 16 besitzt einen Durchgang, durch den hindurch sich das Betätigungselement 11 erstreckt. Das Stellelement 16 ist dabei so am Grundkörper 10 eingerichtet, dass dieses nicht mit einer Gewindeverbindung mit dem Betätigungselement 11 zusammenwirkt.

Am Linsenführungsteil 30 ist eine Eingabeöffnung 33 gezeigt, durch die vor Inbetriebnahme des Injektors 1 ein Viskoelastikum eingegeben werden kann. Dieses benetzt sodann die innenliegende Intraokularlinse und das innere Lumen insbesondere im Linsenführungsteil 30, um den Prozess des Austreibens der Intraokularlinse zu begünstigen beziehungsweise für die Intraokularlinse beschädigungsfrei zu ermöglichen. Der Austrieb der Intraokularlinse erfolgt schließlich über eine Austriebsdüse 31 an der freien Spitze des Linsenführungsteils 30.

Der Injektor 1 weist mit dem Stellelement 16 ein Mittel auf, mit dem für den Gebrauch des Injektors 1 zwischen einer Schubbetätigung und einer Schraubbetätigung umgeschaltet werden kann. Das Stellelement 16 ist für die Umschaltung zwischen der Schubbetätigung und der Schraubbetätigung im Ausführungsbeispiel gemäß Figur 1 um die Betätigungsachse 12 drehbar am Grundkörper 10 ausgebildet, wobei die Wechselwirkung des Stellelementes 16 mit dem Betätigungselement 11 in den weiteren Figuren näher dargestellt wird. Gemäß eines nachfolgenden Ausführungsbeispiels kann das Stellelement 16 auch ohne eine Verdrehung axial in der Betätigungsachse 12 verschoben werden, um den Effekt der Umschaltung zwischen der Schraubbetätigung und der Schubbetätigung ebenso zu erzielen.

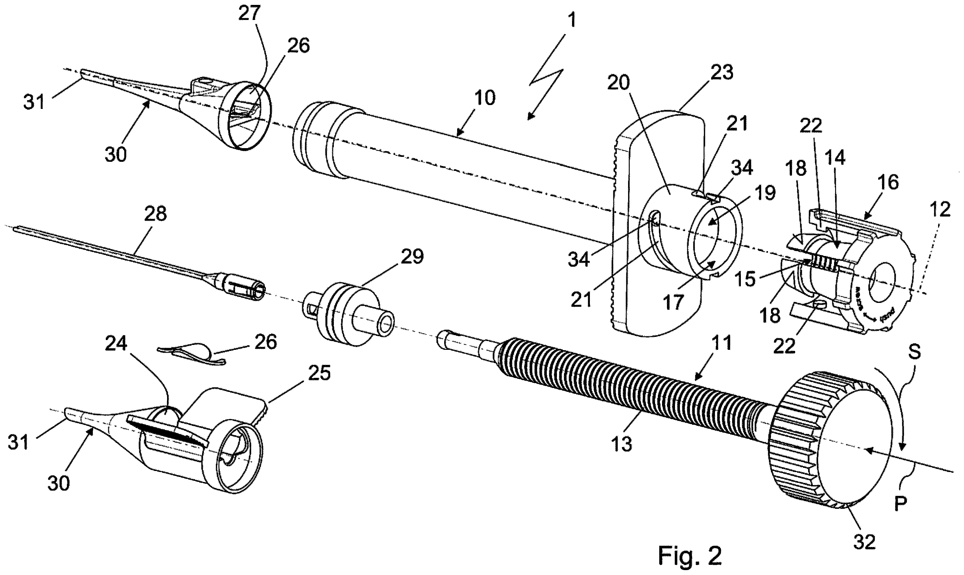

Figur 2 zeigt ein für die Erfindung nicht ausschließliches, aber bevorzugtes Ausführungsbeispiel des Injektors 1 in einer Explosionsdarstellung, in der die wesentlichen Bestandteile des Injektors 1 beispielhaft dargestellt sind, wobei weitere Bestandteile des Injektors 1 vorhanden sind, die nicht dargestellt sind, da diese zur Darlegung der vorliegenden Erfindung nicht notwendig sind, die jedoch auch Bestandteil des erfindungsgemäßen Injektors 1 sein sollen, sodass die Abbildung nicht abschließend zu verstehen ist, womit insbesondere Mittel zur Einwirkung auf die Intraokularlinse zu verstehen sind.

Die Darstellung zeigt den Grundkörper 10 vereinzelt, und ein erstes fluchtend mit der Betätigungsachse 12 dargestelltes Linsenführungsteil 30 ist mit einer Aufnahmekammer 27 ausgeführt, in der eine Intraokularlinse 26 eingelegt ist (sichtbar anhand einer der Haptiken), und ein unterseitig dargestelltes alternativ einsetzbares zweites Linsenführungsteil 30 bildet eine alternative Ausgestaltung zum ersten Linsenführungsteil 30 und weist ein Aufnahmemittel 24 zur Aufnahme einer Linsenkartusche 25 auf, in der die vereinzelt dargestellte Intraokularlinse 26 eingelegt ist. Wird die Intraokularlinse 26 in die Linsenkartusche 25 eingelegt und wird die Linsenkartusche 25 sodann in das Aufnahmemittel 24 eingesetzt, kann die Intraokularlinse 26 mit einem Kolben 28 vorderseitig aus der Austriebsdüse 31 in die hintere Augenkammer des menschlichen oder tierischen Auges ausgetrieben werden. Ist der Injektor 1 als Pre-Load System ausgeführt, kann das oberseitig dargestellte Linsenführungsteil 30 Anwendung finden, das die Aufnahmekammer 27 aufweist, in der die Intraokularlinse 26 bereits herstellerseitig eingelegt werden kann, um das Pre-Load System als einsatzfertige Einheit bereits mit der Intraokularlinse kommerziell bereitzustellen.

Zum Austreiben der Intraokularlinse 26 führt der Kolben 28 eine Linearbewegung in der Betätigungsachse 12 aus, und wird das Betätigungselement 11 im Betriebsmodus der Schraubbetätigung S in den Grundkörper 10 eingedreht, so dient ein Drehübertrager 29 dafür, dass die Rotationsbewegung des Betätigungselementes 11 nicht auf den Kolben 25 übertragen wird.

Wird nicht das Linsenführungsteil 30 mit der direkt in der Aufnahmekammer 27 aufgenommenen Intraokularlinse 26 genutzt, kann alternativ das Linsenführungsteil 30 Anwendung finden, das mit dem Aufnahmemittel 24 ausgestattet ist, in das die Linsenkartusche 25 mit einer in dieser eingebrachten Intraokularlinse 26 eingesetzt werden kann. Dabei bildet das Linsenführungsteil 30 mit der Aufnahmekammer 27 das Pre-Load-System, gemäß dem der Injektor 1 mit der bereits werksseitig eingelegten Intraokularlinse 26 verkauft wird. Alternativ kann der Injektor 1 auch ohne voreingelegte Intraokularlinse 26 angeboten werden, wofür das Linsenführungsteil 30 das Aufnahmemittel 24 aufweist, in das die Linsenkartusche 25 mit der Intraokularlinse 26 erst kurz vor Gebrauch des Injektors 1 eingesetzt werden kann, was als ein sogenanntes Kartuschensystem bezeichnet wird. Das Aufnahmemittel 24 zur Aufnahme einer Linsenkartusche und/oder die Aufnahmekammer 27 zur direkten Aufnahme einer Intraokularlinse 26 können anstatt im Linsenführungsteil 30 alternativ auch im Grundkörper 10 eingerichtet sein, oder das Aufnahmemittel 24 oder die Aufnahmekammer 27 sind in einem räumlichen Bereich zwischen dem Linsenführungsteil 30 und dem Grundkörper 10 ausgebildet, wenn das Linsenführungsteil 30 am Grundkörper 10 angeordnet wurde.

Hinter dem Flügelgriff 23 besitzt der Grundkörper 10 in Gestalt einer Anformung einen Aufnahmeabschnitt 20, auf den das Stellelement 16 um die Betätigungsachse 12 verdrehbar und/oder axial verschiebbar aufgebracht ist. Hierbei weist die Außenoberfläche des Aufnahmeabschnittes 20 eine Nut 21 auf, die spiralförmig über dem Aufnahmeabschnitt 20 in diesen eingebracht ist, sodass bei einer Verdrehung des Stellelementes 16 um die Betätigungsachse 12 das Stellelement 16 zugleich eine Bewegung in der Betätigungsachse 12 ausführt. An den Endabschnitten der Nut 21 können Rastmittel 34 eingebracht sein, und wird das Stellelement 16 in die jeweilige Endlage verdreht, gibt es für den Benutzer eine haptische Rückmeldung und das Stellelement 16 verharrt selbsttätig in der Endstellung, was wichtig ist, da über die Federarme 14 bei einer Schraubbetätigung eine Axialkraft in das Stellelement 16 eingebracht werden muss. Innenseitig im Stellelement 16 sind zur Wirkverbindung mit der Nut 21 Zapfen eingebracht, die in die Nut 21 eingreifen und in der gezeigten Figur lediglich nicht dargestellt sind.

Der Aufnahmeabschnitt 20 ist hülsenförmig ausgeführt und insbesondere einteilig mit dem Grundkörper 10 rückseitig des Flügelgriffes 23 verbunden und der Aufnahmeabschnitt 20 bildet einen Bestandteil des Grundkörper 10.

Innerhalb des Aufnahmeabschnittes 20 ist eine um die Betätigungsachse 12 umlaufende Innenfläche 17 ausgebildet, die gemäß dem gezeigten Ausführungsbeispiel einen Innenkegel 19 bildet, der einen sich in Richtung zur offenen Seite des Aufnahmeabschnittes 20 aufweitenden Durchmesser aufweist. Die Innenfläche 17 ist als eine Oberfläche innenseitig am Grundkörper 10 ausgebildet dargestellt. Da der Grundkörper 10 aber auch mehrteilig ausgebildet sein kann, kann die Innenfläche 17 auch an einem Einzelteil, zum Beispiel in Form eines einsetzbaren Ringes, ausgeführt sein, das bzw. der im Sinne der Erfindung dennoch zum Grundkörper 10 gehört und einen Bestandteil desselben bildet.

Am Stellelement 16 sind mehrere Federarme 14 angeordnet, die durch eine elastische Verformung in Richtung zur Betätigungsachse 12 nach innen oder von der Betätigungsachse 12 selbsttätig federnd wieder weg nach außen bewegt werden können. In einer nicht kraftbeaufschlagten Anordnung der Federarme 14 weisen diese einen Abstand zueinander bzw. zur Betätigungsachse 12 auf, gemäß dem das Außengewinde 13 auf dem Betätigungselement 11 mittig zwischen den Federarmen 14 axial frei bewegt werden kann. Der Auslieferzustand des Injektors 1 sollte daher in einer Stellung des Stellelementes 16 eingerichtet werden, in der dieses in der Stellposition für die Schubbetätigung P steht, sodass die Federarme 14 nicht dauerhaft kraftbeaufschlagt sind und ihre Rückfederwirkung über eine längere Lagerzeit beibehalten.

Durch eine axiale Verlagerung des Stellelementes 16 in der Betätigungsachse 12 wird eine elastische Federbewegung der Federarme 14 in Richtung zur mittigen Betätigungsachse 12 erzeugt. Insbesondere wenn das Stellelement 16 hin zum Flügelgriff 23 verlagert wird, vorzugsweise durch eine Verdrehung, wird durch die Führung der Nut 21 die Verlagerung des Stellelementes 16 in der Betätigungsachse 12 in Richtung zum Flügelgriff 23 bewirkt. Durch den Kontakt der Außenflächen 18 der Federarme 14 mit der kegelförmigen Innenfläche 17 im Grundkörper 10 federn die Federarme 14 elastisch nach innen zur Betätigungsachse 12 hin ein, wenn das Stellelement 16 in Richtung zum Flügelgriff 23 verlagert wird, sodass eine Gewindeeingriffsstruktur 15 auf der Innenseite der Federarme 14, hier nur indirekt gestrichelt dargestellt, mit dem Außengewinde 13 des Betätigungselementes 11 in Eingriff gelangen kann, um sodann den Injektor 1 für die Schraubbetätigung S einzurichten.

Federn die Federarme 14 bei einer axialen Verlagerung des Stellelementes 16 vom Flügelgriff 23 weg wieder radial nach außen auf, insbesondere durch eine Rückdrehung, indem die Außenflächen 18 der Federarme 14 in den Bereich des größeren Durchmessers der Innenfläche17 mit dieser in Kontakt gelangen, so kann die Schubbetätigung P des Injektors 1 eingerichtet werden, da sich die Federarme 14 nicht mehr mit dem Außengewinde 13 des Betätigungselementes 11 in Eingriff befinden.

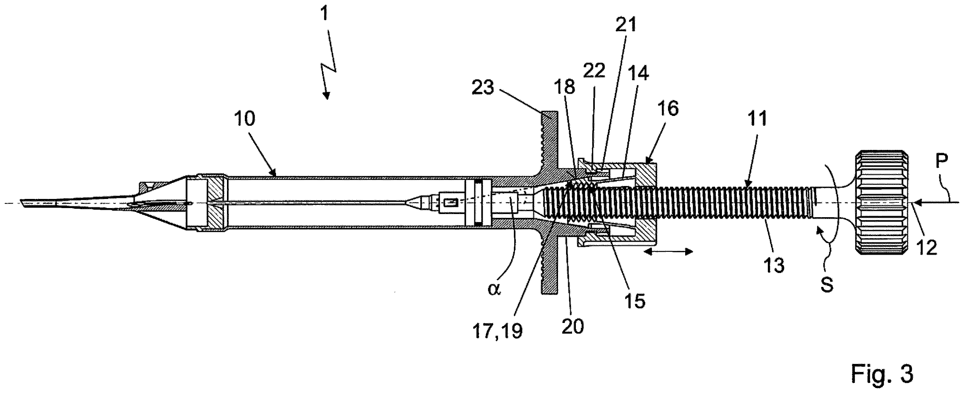

Fig. 3 zeigt ein erstes Ausführungsbeispiel des Injektors 1 in einer Querschnittsansicht. Dargestellt sind der Grundkörper 10 mit dem Flügelgriff 23, und rückseitig am Flügelgriff 23 befindet sich ein Aufnahmeabschnitt 20, der einteilig mit dem Grundkörper 10 ausgebildet ist und sich mit dem Grundkörper 10 gemeinsam in der Betätigungsachse 12 erstreckt. Auf dem Aufnahmeabschnitt 20 ist das Stellelement 16 aufgenommen, und rückseitig ist in den Grundkörper 10 das Betätigungselement 11 mit dem Außengewinde 13 anteilig eingeführt.

In der Außenoberfläche des Aufnahmeabschnittes 20 ist eine Nut 21 eingebracht, und innenseitig im Stellelement 16 befindet sich ein Zapfen 22, der in die Nut 21 hineinragt und in dieser geführt ist. Wird das Stellelement 16 gemäß dem gezeigten Drehpfeil verdreht, kann dieses axial verlagert werden entlang der Betätigungsachse 12, wie mit einem Doppelpfeil angedeutet. Wird das Stellelement 16 also in einer ersten Drehrichtung um die Betätigungsachse 12 verdreht, so nähert sich das Stellelement 16 beispielsweise dem Flügelgriff 23 an, indem sich dieses entlang der Betätigungsachse 12 nach links verlagert. Wird das Stellelement 16 in eine entgegengesetzte Richtung verdreht, so vergrößert sich der Abstand zum Flügelgriff 23 wieder, und das Stellelement 16 verlagert sich entlang der Betätigungsachse 12 nach rechts.

Innenseitig im Stellelement 16 sind Federarme 14 angeformt, die auf besondere Weise mit dem Grundkörper 10 in Wechselwirkung stehen. Innerhalb des Aufnahmeabschnittes 20 ist am Grundkörper 10 eine Innenfläche 17 ausgestaltet, die sich insbesondere rotationssymmetrisch um die Betätigungsachse 12 herum erstreckt. Die Innenfläche 17 ist so ausgestaltet, dass sich über der Innenfläche 17 ein Innenkegel 19 ergibt. Der Innenkegel 19 ist damit so innerhalb des Aufnahmeabschnittes 20 ausgestaltet, dass sich der Kegel in Richtung zur freien Öffnung des Aufnahmeabschnittes 20 aufweitet. Das Beispiel zeigt dabei die Innenfläche 17 im Bereich des Aufnahmeabschnittes 20, diese kann aber auch weiter innenliegend im Grundkörper 10 eingebracht sein, beispielsweise in oder auf der Höhe des Flügelgriffs 23.

Das Stellelement 16 umgreift den Aufnahmeabschnitt 20, und die Federarme 14 weisen Außenflächen 18 auf, die mit der Innenfläche 17 in Wirkkontakt stehen. Durch die unter dem Winkel α ausgebildete Schräge der Innenfläche 17 ergibt sich ein radiales Einfedern der Federarme 14 zur Betätigungsachse 12 hin, wenn das Stellelement 16 in Richtung zum Flügelgriff 23 verlagert wird. Wird das Stellelement 16 wieder so verlagert, dass der Abstand zum Flügelgriff 23 zunimmt, so federn die Federarme 14 wieder auf und der Abstand zur Betätigungsachse 12 vergrößert sich wieder.

Die Federarme 14 weisen innenseitig jeweils eine Gewindeeingriffsstruktur 15 auf, die mit dem Außengewinde 13 des Betätigungselementes 11 in Eingriff gelangen, wenn das Stellelement 16 durch eine Verdrehung in Richtung zum Flügelgriff 23 verlagert wird. Dabei wandern die Außenflächen 18 an der Innenfläche 17 entlang und die Federarme 14 werden infolge der Form des Innenkegels 19 radial nach innen gedrückt. Befinden sich die Federarme 14 durch die Einfederung der Federarme 14 nach innen in Eingriff mit dem Außengewinde 13, so kann der Injektor 1 mit der Schraubbetätigung S betätigt werden. Dabei bilden die Gewindeeingriffsstrukturen 15 auf der Innenseite der Federarme 14 eine Art Innengewinde oder Gewindedurchgang für das Außengewinde 13 des Betätigungselementes 11, sodass das Betätigungselement 11 in ganz gewöhnlicher Weise in den Grundkörper 10 eingeschraubt werden kann. Dabei genügen ein oder zwei Gewindesegmente als Gewindeeingriffsstruktur 15 und folglich genügen ein oder zwei Federarme 14.

Wird das Stellelement 16 durch Rückdrehung wieder nach rechts verlagert, sodass der Abstand zum Flügelgriff 23 zunimmt, so können die Federarme 14 elastisch auffedern, indem die Außenflächen 18 auf einen größeren Durchmesser der Innenfläche 17 in Gestalt des Innenkegels 19 unter Einhaltung des Wirkkontaktes übergehen. Dadurch gelangen die Gewindeeingriffsstrukturen 15 auf der Innenseite der Federarme 14 außer Eingriff mit dem Außengewinde 13 des Betätigungselementes 11, und das Betätigungselement 11 kann axial in der Betätigungsachse 12 verlagert werden, ohne dass eine Schraubbewegung in das Betätigungselement 11 eingeleitet werden muss. Damit kann der Injektor 1 mit der Schubbetätigung P bedient werden.

Damit die axiale Verlagerung des Stellelementes 16 entlang der Betätigungsachse 12 erzeugt wird, kann die Nut 21 schraubenartig oder spiralartig auf der Außenumfangsfläche des Aufnahmeabschnittes 20 ausgebildet sein. Hierfür genügt eine Nut 21, wobei zur besseren Führung und Stabilisierung des Stellelementes 16 auf dem Aufnahmeabschnitt 20 auch zwei oder mehrere Nuten 21 vorgesehen sein können, die einen jeweils gleichen, etwa spiralförmigen oder S-förmigen Verlauf um die Betätigungsachse 12 mit einer Steigung aufweisen können, um die Axialbewegung im Stellelement 16 zu erzeugen.

Fig. 3 zeigt ein zweites Ausführungsbeispiel des Injektors 1 in einer Querschnittsansicht mit einer abgeänderten Innenfläche 17, die über den Umfang einen veränderlichen Innenradius Ri aufweist. Durch den veränderlichen Innenradius Ri kann durch eine Verdrehung des Stellelementes 16 ebenfalls eine elastische Verformung der Federarme 14 zur Betätigungsachse 12 hin und von dieser weg erzeugt werden, indem das Stellelement 16 ohne axiale Verlagerung in einfacher Weise auf dem Aufnahmeabschnitt 20 verdreht wird. Hierfür kann die Nut 21 in Umfangsrichtung verlaufen, ohne eine Steigung zu besitzen, sodass bei einer Verdrehung des Stellelementes 16 dieses einen konstanten Abstand zum Flügelgriff 23 wahrt. Zur Führung des Stellelementes 16 dient wiederum wenigstens ein Zapfen 22 auf dessen Innenseite, der in der Nut 21 verläuft. In den Endlagen der rotatorischen Verstellung des Stellelementes 16 können Raststufen in der Nut 21 eingebracht sein, um eine angenehme haptische Rückmeldung für den Benutzer zu erhalten, dass die jeweilige Endposition erreicht ist, die die Schubbetätigung oder die Schraubbetätigung repräsentiert.

Befindet sich das Stellelement 16 in einer Drehposition, in der die Federarme 14 radial nach innen einfedern, so gelangen auch hier die Gewindeeingriffsstrukturen 15 auf der Innenseite der Federarme 14 in Eingriff mit dem Außengewinde 13 des Betätigungselementes 11. Bei einer Rückdrehung des Stellelementes 16 um die Betätigungsachse 12 federn die Federarme 14 eigenelastisch wieder auf, sodass die Gewindeeingriffsstrukturen 15 wieder außer Eingriff mit dem Außengewinde 13 des Betätigungselementes 11 gelangen.

Die Innenfläche 17 mit dem über dem Umfang oder in der Umfangsrichtung sich ändernden Innenradius Ri ist innenseitig im Aufnahmeabschnitt 20 lediglich beispielhaft gezeigt, und die Innenfläche 17 kann auch innerhalb des Flügelgriffs 23 oder weiter vorderseitig im Grundkörper 10 in dessen Durchgang eingebracht sein. Die Innenfläche 17 kann dabei auch an einem Einzelteil des Grundkörpers 10 angebracht sein, beispielsweise an einem entsprechenden Ringelement, das im Sinne der Erfindung ebenfalls Bestandteil des Grundkörpers 10 ist.

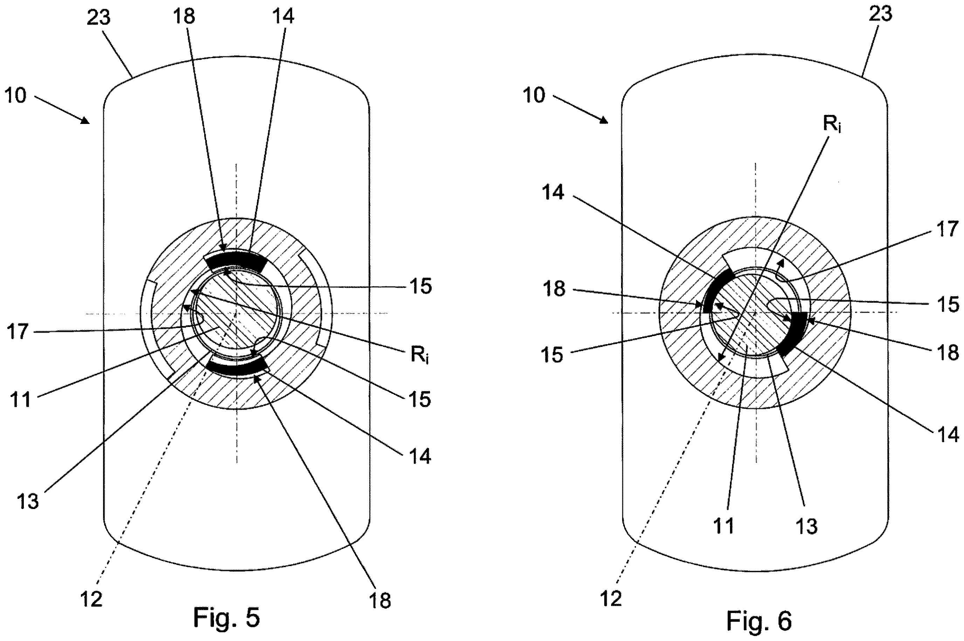

Die Figuren 5 und 6 zeigen eine Querschnittsansicht durch den Grundkörper 10, dargestellt durch den Flügelgriff 23. In den inneren Durchgang im Grundkörper 10 erstreckt sich das Betätigungselement 11 hinein, das geschnitten dargestellt ist, sodass die Betätigungsachse 12 senkrecht auf der Ebene der Abbildung steht. Durch den Querschnitt sind die Federarme 14 zumindest teilweise zu sehen, die sich zwischen der Innenfläche 17 im Grundkörper 10 und dem Betätigungselement 11 befinden. In Figur 5 befinden sich die Federarme 14 radial beanstandet zu dem Betätigungselement 11 und in Figur 6 greifen die Federarme 14 in das Außengewinde 13 des Betätigungselement 11 ein.

Die gezeigte Innenfläche 17 mit einem über dem Umfang veränderlichen Innenradius Ri zeigt eine mögliche Variante, eine radiale Verstellung der Federarme 14 zu erzeugen, indem die Federarme 14 in verschiedene Umfangspositionen mit der Innenfläche 17 gebracht werden.

Figur 5 zeigt die Stellposition der Federarme 14, in der die Schubbetätigung ermöglicht ist, und Figur 6 zeigt eine Stellposition der Federarme 14, in der die Schraubbetätigung ermöglicht ist.

Zur Einstellung der Schubbetätigung befinden sich die Federarme 14 über ihre außenseitigen Außenflächen 18 an einer Umfangsposition in Kontakt mit der Innenoberfläche 17, in der der Innenradius Ri besonders groß ist. Durch das eigenelastische Auffedern der Federarme 14 gelangt die Gewindeeingriffsstruktur 15 außer Eingriff mit dem Außengewinde 13 auf dem Betätigungselement 11.

Wie in Figur 6 gezeigt, können die Federarme 14 durch eine Verdrehung des nicht gezeigten Stellelementes gegen den Uhrzeigersinn in eine Stellposition verdreht werden, in der die Schraubbetätigung S ermöglicht wird. In dieser Position befindet sich die Außenfläche 18 des Federarms 14 an einer Umfangsposition in Kontakt mit der Innenoberfläche 17, in der der Innenradius Ri besonders klein ist, und die Gewindeeingriffsstruktur 15 innenseitig in den Federarmen 14 wird in Eingriff mit dem Außengewinde 13 auf dem Betätigungselement 11 gebracht. In dieser Position kann das Betätigungselement 11 nur durch eine Schraubbewegung in den Grundkörper eingeschraubt werden.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch macht. Sämtliche aus den Ansprüchen, der Beschreibung oder den Zeichnungen hervorgehenden Merkmale undloder Vorteile, einschließlich konstruktiver Einzelheiten oder räumlicher Anordnungen, können sowohl für sich als auch in den verschiedensten Kombinationen erfindungswesentlich sein.

### Bezugszeichenliste:

- 1: Injektor

- 10: Grundkörper
- 11: Betätigungselement
- 12: Betätigungsachse
- 13: Außengewinde
- 14: Federarm
- 15: Gewindeeingriffsstruktur
- 16: Stellelement
- 17: Innenfläche
- 18: Außenfläche
- 19: Innenkegel
- 20: Aufnahmeabschnitt
- 21: Nut
- 22: Zapfen
- 23: Flügelgriff
- 24: Aufnahmemittel
- 25: Linsenkartusche
- 26: Intraokularlinse
- 27: Aufnahmekammer
- 28: Kolben
- 29: Drehübertrager
- 30: Linsenführungsteil
- 31: Austriebsdüse
- 32: Griffstück
- 33: Eingabeöffnung
- 34: Rastmittel
- P: Schubbetätigung
- S: Schraubbetätigung
- α: Winkel
- Ri: Innenradius

## Patentansprüche

1. Injektor (1) zur Implantation einer Intraokularlinse, aufweisend einen Grundkörper (10), in den rückseitig ein länglich ausgebildetes Betätigungselement (11) abschnittsweise hineinragt und in einer Betätigungsachse (12) beweglich geführt ist, und wobei eine Bewegung des Betätigungselementes (11) in den Grundkörper (10) wahlweise mittels einer Schubbetätigung (P) entlang der Betätigungsachse (12) oder mittels einer Schraubbetätigung (S) um die Betätigungsachse (12) erzeugbar ist, und wobei das Betätigungselement (11) wenigstens auf einem Abschnitt ein Außengewinde (13) aufweist, wobei am Grundkörper (10) ein Stellelement (16) um die Betätigungsachse (12) verdrehbar oder in der Betätigungsachse (12) verschiebbar angeordnet ist, wobei das Stellelement (16) wenigstens einen Federarm (14) aufweist, der an einer zum Außengewinde (13) weisenden Innenfläche eine Gewindeeingriffsstruktur (15) aufweist, wobei am Grundkörper (10) eine Innenfläche (17) ausgebildet ist, die mit einer Außenfläche (18) am Federarm (14) einen Wirkkontakt bildet, wobei die Innenfläche (17) und/oder die Außenfläche (18) unter einem Winkel (α) zur Betätigungsachse (12) geneigt ausgebildet ist oder wobei die Innenfläche (17) in einer Umfangsrichtung um die Betätigungsachse (12) wenigstens abschnittsweise einen sich ändernden Innenradius (Ri) aufweist, sodass die Verdrehung oder die Verschiebung des Stellelementes (16) eine elastische Verformung des Federarms (14) zur Betätigungsachse (12) hin und von der Betätigungsachse (12) weg erzeugbar ist und sodass die Gewindeeingriffsstruktur (15) mit dem Außengewinde (13) wahlweise in Eingriff oder außer Eingriff bringbar ist.

2. Injektor (1) nach Anspruch 1,
**dadurch gekennzeichnet,**
**dass** das Stellelement (16) als Schiebeelement ausgeführt und im oder am Grundkörper (10) beweglich angeordnet ist und/oder dass das Stellelement (16) ringförmig oder hülsenförmig ausgeführt ist und das Betätigungselement (11) teilumfänglich oder vollumfänglich umschließt und am Grundkörper (10) verschiebbar oder verdrehbar ist.

3. Injektor (1) nach einem der Ansprüche 1 oder 2,
**dadurch gekennzeichnet,**
**dass** der Grundkörper (10) einen Aufnahmeabschnitt (20) mit einem Durchgang aufweist, durch den sich das Betätigungselement (11) hindurch erstreckt und wobei das Stellelement (16) am oder auf dem Aufnahmeabschnitt (20) entlang der Betätigungsachse (12) axial beweglich undloder um die Betätigungsachse (12) drehbeweglich aufgenommen ist.

4. Injektor (1) nach Anspruch 3,
**dadurch gekennzeichnet,**
**dass** die Innenfläche (17) innenseitig im Aufnahmeabschnitt (20) des Grundkörpers (10) ausgebildet ist.

5. Injektor (1) nach einem der vorgenannten Ansprüche,
**dadurch gekennzeichnet,**
**dass** der wenigstens eine Federarm (14) so am Stellelement (16) angeordnet ist, dass sich der Federarm (14) zumindest abschnittsweise zwischen das Betätigungselement (11) und der Innenfläche (17) im Grundkörper (10) hinein erstreckt und/oder sich in den Aufnahmeabschnitt (20) hinein erstreckt.

6. Injektor (1) nach einem der vorgenannten Ansprüche,
**dadurch gekennzeichnet,**
**dass** die Innenfläche (17) wenigstens abschnittsweise einen Innenkegel (19) im Grundkörper (10) bildet.

7. Injektor (1) nach einem der Ansprüche 1 bis 5,
**dadurch gekennzeichnet,**
**dass** die Innenoberfläche (17) mit dem sich in Umfangsrichtung ändernden Innenradius (Ri) auf einem Umfangssegment (U1, U2) um die Betätigungsachse (12) im Grundköper (10) ausgebildet ist, wobei das Umfangssegment (U1, U2) sich auf einem Umfang mit einem Umfangswinkel von 360° oder auf einem Umfangswinkel von 180° oder auf einem Umfangswinkel von 120° oder auf einem Umfangswinkel von 90° erstreckt.

8. Injektor (1) nach einem der vorgenannten Ansprüche,
**dadurch gekennzeichnet,**
**dass** wenigstens ein Federarm (14), zwei sich gegenüberstehende Federarme (14), drei, vier oder mehr Federarme (14) am Stellelement (16) angeordnet sind.

9. Injektor (1) nach einem der vorgenannten Ansprüche,
**dadurch gekennzeichnet,**
**dass** zwischen dem Aufnahmeabschnitt (20) und dem Stellelement (13) wenigstens eine Nut (21) und ein Zapfen (22) ausgebildet sind, wobei der Zapfen (22) in der Nut (21) geführt ist.

10. Injektor (1) nach einem der vorgenannten Ansprüche,
**dadurch gekennzeichnet,**
**dass** das Stellelement (16) mittels einer Gewindeverbindung am Aufnahmeabschnitt (20) angeordnet ist, sodass mit einer Verdrehung des Stellelementes (16) um die Betätigungsachse (12) das Stellelement (16) eine Bewegung entlang der Betätigungsachse (12) ausführt.

11. Injektor (1) nach einem der vorgenannten Ansprüche,
**dadurch gekennzeichnet,**
**dass** der Grundkörper (10) einen Flügelgriff (23) aufweist, wobei sich der Aufnahmeabschnitt (20) rückseitig des Flügelgriffes (23) als Fortsatz anschließt.

12. Injektor (1) nach einem der vorgenannten Ansprüche,
**dadurch gekennzeichnet,**
**dass** der Injektor (1) ein Aufnahmemittel (24) für eine Linsenkartusche (25) aufweist, wobei in die Linsenkartusche (25) eine Intraokularlinse (26) eingebracht ist und wobei die Linsenkartusche (25) mit der Intraokularlinse (26) in das Aufnahmemittel (24) einsetzbar ist.

13. Injektor (1) nach einem der Ansprüche 1 bis 12,
**dadurch gekennzeichnet,**
**dass** der Injektor (1) eine im Grundkörper (10) undloder in einem vorderseitig am Grundkörper (10) angeordneten Linsenführungsteil (30) ausgebildete Aufnahmekammer (27) aufweist, in der eine Intraokularlinse (26) eingelegt ist, sodass der Injektor (1) mit der eingelegten Intraokularlinse (26) eine einzeln handhabbare und handelbare Einheit bildet.

14. Injektor (1) nach einem der vorgenannten Ansprüche,
**dadurch gekennzeichnet,**
**dass** im Grundkörper (10) ein Kolben (28) aufgenommen ist, der über einen Drehübertrager (29) mit dem Betätigungselement (11) axial verschiebbar ist.

## Claims

1. Injector (1) for implantation of an intraocular lens (26), having a base body (10) and an elongated actuation element (11), which projects at least partly into a rear section of the base body (10) and which is movably guided in an actuation axis (12), and wherein a movement of the actuation element (11) into the base body (10) can be generated selectively by means of a push actuation (P) along the actuation axis (12) or by means of a screw actuation (S) about the actuation axis (12), and wherein the actuation element (11) features an external thread (13) at least on one section, wherein a switch element (16) is arranged to the base body (10), whereas the switch element (16) is rotatable about the actuation axis (12) or is displaceable in the actuation axis (12), whereas the switch element (16) features at least one spring arm (14), having a thread engagement structure (15) on an inner surface facing the external thread (13), whereas at the base body (10) is formed an inner surface (17), which forms an operative contact with an outer surface (18) of the spring arm (14), whereas the inner surface (17) and/or the outer surface (18) is formed inclined to the actuation axis (12) at an angle (α) or the inner surface (17) features an inner radius (Ri) varying at least in sections in a circumferential direction about the actuation axis (12), so that upon rotation or upon displacement of the switch element (16) an elastic deformation of the spring arm (14) towards the actuation axis (12) and away from the actuation axis (12) can be generated, whereby the thread engagement structure (15) can be selectively engaged or disengaged with the external thread (13).

2. Injector (1) according to claim 1,
**characterized in,**
**that** the switch element (16) is designed as a displacement element and is arranged movably in or on the base body (10) and/or in that the switch element (16) is designed ring-shaped or hull-shaped and encompasses the actuation element (11) partially or completely and is displaceable or rotatable arranged to or on the base body (10).

3. Injector (1) according to one of claims 1 or 2,
**characterized in,**
**that** the base body (10) has a receiving section (20) with a passage, through which the actuation element (11) extends, and wherein the switch element (16) is received at or on the receiving section (20) so as to be axially movable along the actuation axis (12) and/or rotatable about the actuation axis (12).

4. Injector (1) according to claim 3,
**characterized in,**
**that** the inner surface (17) is formed on the inside of the receiving section (20) of the base body (10).

5. Injector (1) according to one of the aforementioned claims,
**characterized in**
**that** the at least one spring arm (14) is arranged to the switch element (16) in such a way that the spring arm (14) extends at least partly between the actuation element (11) and the inner surface (17) in the base body (10) and/or extends into an inner hole of the receiving section (20).

6. Injector (1) according to one of the aforementioned claims,
**characterized in,**
**that** the inner surface (17) forms at least partly an inner cone (19) in the base body (10).

7. Injector (1) according to one of claims 1 to 5,
**characterized in,**
**that** the inner surface (17) with the inner radius (Ri) varying in a circumferential direction is formed on a circumferential segment (U1, U2) about the actuation axis (12) in the base body (10), wherein the circumferential segment (U1, U2) extends on a circumference with a circumferential angle of 360° or with a circumferential angle of 180° or with a circumferential angle of 120° or with a circumferential angle of 90°.

8. Injector (1) according to one of the aforementioned claims,
**characterized in,**
**that** at least one spring arm (14), two spring arms (14) facing each other, three, four or more spring arms (14) are arranged on the switch element (16).

9. Injector (1) according to one of the aforementioned claims,
**characterized in,**
**that** at least one groove (21) and one pin (22) are formed between the receiving section (20) and the switch element (16), wherein the pin (22) is guided in the groove (21).

10. Injector (1) according to one of the aforementioned claims,
**characterized in,**
**that** the switch element (16) is arranged on the receiving section (20) by means of a threaded connection, so that upon rotation of the switch element (16) about the actuation axis (12), the switch element (16) executes a movement along the actuation axis (12).

11. Injector (1) according to one of the aforementioned claims,
**characterized in,**
**that** the base body (10) has a wing handle (23), wherein the receiving section (20) adjoins the rear side of the wing handle (23) as an extension of the base body (10).

12. Injector (1) according to one of the aforementioned claims,
**characterized in,**
**that** the injector (1) has a receiving means (24) for a lens cartridge (25), wherein an intraocular lens (26) is inserted into the lens cartridge (25), and wherein the lens cartridge (25) with the inner intraocular lens (26) can be inserted into the receiving means (24).

13. Injector (1) according to one of claims 1 to 11,
**characterized in,**
**that** the injector (1) has a receiving chamber (27), which is formed in the base body (10) and/or in a lens guide section (30) arranged at the front end of the base body (10), and in which an intraocular lens (26) is inserted, so that the injector (1) forms an individually manageable and tradable unit with the inserted intraocular lens (26).

14. Injector (1) according to one of the aforementioned claims,
**characterized in,**
**that** a piston (28) is received in the base body (10) and can be displaced axially by the actuation element (11) via a rotary joint (29).

## Revendications

1. Injecteur (1) destiné à l'implantation d'une lentille intraoculaire, présentant un corps de base (10), dans lequel, sur le côté arrière, un élément d'actionnement (11) réalisé allongé dépasse en partie et est guidé mobile dans un axe d'actionnement (12), et dans lequel un déplacement de l'élément d'actionnement (11) dans le corps de base (10) peut être généré au choix au moyen d'un actionnement par coulissement (P) le long de l'axe d'actionnement (12) ou au moyen d'un actionnement par vissage (S) autour de l'axe d'actionnement (12), et dans lequel l'élément d'actionnement (11) présente, au moins sur une partie, un filetage extérieur (13),
dans lequel un élément de réglage (16) est agencé sur le corps de base (10) de manière rotative autour de l'axe d'actionnement (12) ou coulissante dans l'axe d'actionnement (12), l'élément de réglage (16) présentant au moins un bras ressort (14), qui présente, sur une face interne orientée vers le filetage extérieur (13), une structure de mise en prise par vissage (15), une face interne (17) étant formée sur le corps de base (10), laquelle forme un contact fonctionnel avec une face externe (18) sur le bras ressort (14), la face interne (17) et/ou la face externe (18) étant réalisées inclinées selon un angle (α) par rapport à l'axe d'actionnement (12) ou la face interne (17) présentant, dans une direction circonférentielle autour de l'axe d'actionnement (12), au moins sur certaines parties, un rayon intérieur (Ri) variable, de telle sorte que la rotation ou le coulissement de l'élément de réglage (16) peut provoquer une déformation élastique du bras ressort (14) vers l'axe d'actionnement (12) et à l'opposé de l'axe d'actionnement (12) et de telle sorte que la structure de mise en prise par vissage (15) peut au choix être amenée en prise avec le filetage extérieur (13) ou séparée de celui-ci.

2. Injecteur (1) selon la revendication 1,
**caractérisé en ce que**
l'élément de réglage (16) est conçu sous la forme d'un élément coulissant et est agencé mobile dans ou sur le corps de base (10) et/ou **en ce que** l'élément de réglage (16) est conçu en forme d'anneau ou en forme de douille et entoure l'élément d'actionnement (11) sur une partie ou sur la totalité de sa circonférence et peut coulisser ou tourner sur le corps de base (10).

3. Injecteur (1) selon l'une des revendications 1 ou 2,
**caractérisé en ce que**
le corps de base (10) présente une partie de réception (20) dotée d'un passage à travers lequel s'étend l'élément d'actionnement (11) et dans lequel l'élément de réglage (16) est reçu contre ou sur la partie de réception (20) de manière mobile axialement le long de l'axe d'actionnement (12) et/ou de manière mobile en rotation autour de l'axe d'actionnement (12).

4. Injecteur (1) selon la revendication 3,
**caractérisé en ce que**
la face interne (17) est réalisée sur le côté intérieur dans la partie de réception (20) du corps de base (10).

5. Injecteur (1) selon l'une des revendications précédentes,
**caractérisé en ce que**
l'au moins un bras ressort (14) est agencé sur l'élément de réglage (16) de telle manière que le bras ressort (14) s'étend au moins en partie entre l'élément d'actionnement (11) et la face interne (17) dans le corps de base (10) et/ou s'étend dans la partie de réception (20).

6. Injecteur (1) selon l'une des revendications précédentes,
**caractérisé en ce que**
la face interne (17) forme, au moins sur certaines parties, un cône intérieur (19) dans le corps de base (10).

7. Injecteur (1) selon l'une des revendications 1 à 5,
**caractérisé en ce que**
la face interne (17) avec le rayon intérieur (Ri) variable dans la direction circonférentielle est réalisée sur un segment de circonférence (U1, U2) autour de l'axe d'actionnement (12) dans le corps de base (10), le segment de circonférence (U1, U2) s'étendant sur une circonférence avec un angle inscrit de 360° ou sur un angle inscrit de 180° ou sur un angle inscrit de 120° ou sur un angle inscrit de 90°.

8. Injecteur (1) selon l'une des revendications précédentes,
**caractérisé en ce que**
au moins un bras ressort (14), deux bras ressorts (14) opposés l'un à l'autre, trois, quatre bras ressorts (14) ou plus sont agencés sur l'élément de réglage (16).

9. Injecteur (1) selon l'une des revendications précédentes,
**caractérisé en ce que**
au moins une rainure (21) et un ergot (22) sont réalisés entre la partie de réception (20) et l'élément de réglage (13), l'ergot (22) étant guidé dans la rainure (21).

10. Injecteur (1) selon l'une des revendications précédentes,
**caractérisé en ce que**
l'élément de réglage (16) est agencé sur la partie de réception (20) au moyen d'un assemblage vissé de telle sorte que, lors d'une rotation de l'élément de réglage (16) autour de l'axe d'actionnement (12), l'élément de réglage (16) exécute un déplacement le long de l'axe d'actionnement (12).

11. Injecteur (1) selon l'une des revendications précédentes,
**caractérisé en ce que**
le corps de base (10) présente une poignée papillon (23), la partie de réception (20) étant dans le prolongement de la poignée papillon (23) à l'arrière sous la forme d'un appendice.

12. Injecteur (1) selon l'une des revendications précédentes,
**caractérisé en ce que**
l'injecteur (1) présente un moyen de réception (24) pour une cartouche de lentille (25), une lentille intraoculaire (26) se trouvant dans la cartouche de lentille (25) et la cartouche de lentille (25) pouvant être introduite avec la lentille intraoculaire (26) dans le moyen de réception (24).

13. Injecteur (1) selon l'une des revendications 1 à 12,
**caractérisé en ce que**
l'injecteur (1) présente une chambre de réception (27) réalisée dans le corps de base (10) et/ou dans une pièce de guidage de lentille (30) agencée sur le corps de base (10) à l'avant, chambre de réception dans laquelle une lentille intraoculaire (26) est insérée, de telle sorte que l'injecteur (1) forme, avec la lentille intraoculaire (26) insérée, une unité pouvant être commercialisée et manipulée individuellement.

14. Injecteur (1) selon l'une des revendications précédentes,
**caractérisé en ce que**
un piston (28) est reçu dans le corps de base (10), ledit piston pouvant être déplacé axialement avec l'élément d'actionnement (11) par le biais d'un transmetteur rotatif (29).
